(19) Europäisches Patentamt / European Patent Office / Office européen des brevets

(11) **EP 3 036 591 B1**

(12) **FASCICULE DE BREVET EUROPEEN**

(45) Date de publication et mention
de la délivrance du brevet:
**04.04.2018 Bulletin 2018/14**

(21) Numéro de dépôt: **13766389.4**

(22) Date de dépôt: **22.08.2013**

(51) Int Cl.:
*G05B 23/02* *(2006.01)*  *F25J 3/04* *(2006.01)*
*B01D 3/42* *(2006.01)*  *G01N 33/00* *(2006.01)*
*G05B 13/04* *(2006.01)*  *G05B 17/02* *(2006.01)*
*G06F 11/00* *(2006.01)*  *G06Q 10/04* *(2012.01)*

(86) Numéro de dépôt international:
**PCT/FR2013/051957**

(87) Numéro de publication internationale:
**WO 2015/025087 (26.02.2015 Gazette 2015/08)**

(54) **DETECTION DE DEFAILLANCES DANS LA DETERMINATION DES CONCENTRATIONS DE COMPOSANTS CHIMIQUES DANS UNE COLONNE DE DISTILLATION**

FEHLERERKENNUNG BEIM BESTIMMEN DER KONZENTRATIONEN VON CHEMISCHEN KOMPONENTEN IN EINER DESTILLATIONSKOLONNE

DETECTION OF FAULTS WHEN DETERMINING CONCENTRATIONS OF CHEMICAL COMPONENTS IN A DISTILLATION COLUMN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**29.06.2016 Bulletin 2016/26**

(73) Titulaire: **L'Air Liquide Société Anonyme pour l'Etude et l'Exploitation des Procédés Georges Claude
75007 Paris (FR)**
(72) Inventeurs:
• **AMMOURI, Fouad
F-91300 Massy (FR)**
• **DUDRET, Stéphane
F-75015 Paris (FR)**
• **ROUCHON, Pierre
F-92190 Meudon (FR)**
(74) Mandataire: **Mercey, Fiona Susan
L'Air Liquide SA
Direction de la Propriété Intellectuelle
75, Quai d'Orsay
75321 Paris Cedex 07 (FR)**
(56) Documents cités:
**EP-A1- 1 522 808     EP-A2- 0 508 386
EP-A2- 0 701 186     US-A- 5 313 800**

**Description**

La présente invention concerne la détection de défaillances dans un dispositif et un procédé de détermination des concentrations de composants chimiques, notamment de l'air, dans une colonne de distillation. Elle concerne aussi une unité de séparation d'air correspondante, un programme d'ordinateur pour la mise en oeuvre du procédé ainsi que le support d'enregistrement pour stocker le programme. Par « concentrations », on entend plus précisément ici et dans la suite du texte, « fractions molaires ». Le terme de « concentrations » est conservé dans un objectif de simplification.

L'exploitation des unités de séparation d'air, connues sous l'acronyme anglais ASU (« Air Separation Units »), nécessite la connaissance des concentrations et/ou des températures à l'intérieur des colonnes de distillation formant ces unités.

Il existe sur le marché des capteurs, dits analyseurs de concentrations, qui permettent de mesurer les concentrations des composants chimiques en un endroit déterminé d'une colonne de distillation. Pour permettre une bonne exploitation de la colonne, il convient de s'assurer de la fiabilité de mesures effectuées par les capteurs.

De nombreuses méthodes ont déjà été proposées en ce sens. Elles reposent pour la plupart sur des techniques de réconciliation de données entre les différentes mesures réalisées par les capteurs. Leur approche est généralement limitée aux modes de fonctionnement en régime stabilisé ou, pour les plus pertinentes, en régime transitoire, pourvu qu'il s'agisse alors de conditions de fonctionnement bien identifiées. Elles s'appuient souvent sur des modèles de boîtes noires dont le réalisme, la mise au point, la facilité d'entretien et l'efficacité restent discutables, en particulier lorsqu'elles sont appliquées à des unités industrielles complexes.

Par ailleurs, outre leur coût prohibitif, l'utilisation d'analyseurs de concentration nécessite de prévoir des tuyaux supplémentaires pour prélever des échantillons dans les colonnes.

L'ajout de tuyaux et le perçage ne sont pas souhaités car ils détériorent l'isolation des colonnes. Par conséquent, le nombre d'analyseurs de concentration pouvant être utilisé dans une colonne est limité, rendant ainsi impossible la mesure de concentrations partout le long de la colonne.

Il est ainsi intéressant d'estimer en temps réel les concentrations à tout endroit d'une colonne de distillation en se basant sur un nombre limité de capteurs mis en place, ceci en tenant compte, notamment, des variations de quantité de produit traité par la colonne de distillation.

Certaines solutions ont été développées en ce sens. Elles s'appuient sur des modèles permettant de reconstituer par calcul la concentration des composants le long de la colonne, à partir des mesures relevées par les capteurs. La fiabilité des résultats obtenus dépend bien sûr de la fiabilité du modèle employé mais également de la fiabilité des capteurs. On conçoit de la sorte l'importance d'une détection rapide de toute anomalie de fonctionnement des capteurs.

Différentes approches sont envisageables. Dans une première approche, on intègre dans le processus d'estimation des concentrations un modèle prévoyant les effets d'une défaillance des capteurs. Le modèle fait varier une donnée spécifique, fictive, dont la valeur est fixée à zéro dans des conditions normales de fonctionnement. On émet alors une alerte lorsque la valeur de la donnée fictive s'éloigne trop de zéro. Une autre approche consiste à détecter les variations brusques des valeurs de concentrations obtenues. Ces méthodes sont cependant lourdes en besoin de calculs.

Parallèlement, il est connu un processus d'estimation des concentrations le long de la colonne utilisant un modèle, dit modèle d'onde. Selon ce modèle, les colonnes de distillation sont considérées comme des lits continus le long desquels les profils de concentration se déplacent comme des ondes se propageant selon les flux de gaz et de liquide. Il en résulte des profils de concentration présentant une allure générale en S le long de la colonne. Le modèle d'onde est paramétré avec un paramètre, dit facteur de forme, qui ajuste la forme de l'onde, notamment l'aplanissement de son allure.

Généralement, le facteur de forme est considéré comme constant dans les modèles d'onde existants. Ainsi, avec ce modèle, l'onde glisse dans la colonne tandis que sa forme reste constante. Or, cette hypothèse est fausse lorsque les conditions de fonctionnement changent, particulièrement à une pureté élevée. Pour remédier à ce défaut, il existe des approches correctives liant le facteur de forme aux conditions de fonctionnement en utilisant des modèles inférentiels ou une estimation en ligne qui vont permettre d'adapter l'allure de la courbe.

Bien qu'associé à une autre approche, il a par ailleurs été développé par la demanderesse un procédé utilisant un modèle d'estimation des concentrations exploitant également un paramètre de réglage permettant de prendre en compte des variations de fonctionnement de la colonne.

EP-A-0701186 et US-A-5313800 décrivent un procédé et un dispositif selon le préambule des revendications 1 et 12. La présente invention vise à améliorer la détection d'une défaillance d'un capteur de concentration en s'appuyant sur ce type de modèles.

A cet effet, la présente invention a pour objet un procédé de détermination des concentrations de composants chimiques d'un produit, notamment de l'air, dans une colonne de distillation, notamment à garnissages, procédé dans lequel on met en oeuvre un modèle permettant d'estimer la concentration des composants à partir de mesures effectuées par un ou plusieurs capteurs, ledit modèle exploitant un paramètre de réglage permettant de prendre en compte des variations de fonctionnement de la colonne, caractérisé en ce qu'il comprend une étape dans laquelle on détecte les valeurs dudit paramètre de réglage sortant d'une plage de variations nominale dudit paramètre pour diagnostiquer une défaillance de l'un ou plusieurs desdits capteurs.

L'invention repose sur le constat qu'avec ce type de modèle, tant que les capteurs fonctionnent correctement, le paramètre de réglage varie de façon connue. Par contre, si l'un ou plusieurs des capteurs est défaillant, le paramètre de réglage varie de façon erratique car il tente de prendre en compte une variation de fonctionnement de la colonne qui n'est pas réelle. Il suffit de la sorte de suivre les variations du paramètre de réglage pour mettre en évidence la défaillance. On exploite ainsi l'une des propres variables du modèle d'estimation de la concentration pour déterminer la présence d'une anomalie de fonctionnement des capteurs, ce qui facilite le processus.

Selon différents caractéristiques de l'invention, qui pourront être prises ensemble ou séparément :

- ledit procédé comprend une étape préalable dans laquelle on établit au moins une dite plage de variations nominale dudit paramètre,
- ladite étape préalable comprend une étape d'apprentissage dans laquelle on enregistre tout ou partie des valeurs prises par ledit paramètre de réglage sur au moins une période de temps, dites valeurs de références,
- ladite étape préalable comprend une étape de détermination de ladite plage de variations nominale à partir desdites valeurs de référence,
- ladite étape d'apprentissage est opérée avec ladite colonne de distillation,
- ladite étape préalable comprend une étape de fusion de données provenant de plusieurs colonnes de distillation et ladite étape de détermination de ladite plage de variations nominale exploite ladite fusion de données pour déterminer une plage de variations nominale globale,
- ladite étape préalable comprend une étape de sélection de ladite plage de variations nominale parmi des plages de variations nominales correspondant à tout ou partie desdites installations et/ou la plage de variations nominale globale,
- ledit procédé comprend une étape d'enregistrement de valeurs prises par ledit paramètre de réglage lors d'une excursion dudit paramètre de réglage en dehors de ladite plage de variations nominale, dite initiale, et une étape de modification de la plage de variation nominale initiale en une plage élargie, tenant compte des valeurs prises par le paramètre de réglage en dehors de ladite plage de variations nominale initiale, quand une vérification permet d'établir que la défaillance associée à ladite excursion est un faux positif,
- on prévoit plusieurs plages de variation nominale, chaque plage correspondant à des conditions de fonctionnement données de ladite colonne de distillation et ledit procédé comprend une étape de détermination des conditions de fonctionnement de la colonne de distillation et une étape de sélection de la plage de variations nominale correspondante,
- ladite unité de distillation comprend plusieurs zones exploitant chacune un dit paramètre de réglage et on utilise une dite plage de variations nominale pour chacune desdites zones.

Cela étant, selon un aspect de l'invention, le modèle employé est du type permettant d'estimer la concentration des composants en fonction du temps et de la position le long de l'axe longitudinal de la colonne.

Plus précisément, il pourra s'agir d'un modèle exploitant un terme de propagation en relation avec une convection desdits composants le long de la colonne et un terme de diffusion axiale en relation avec une diffusion desdits composants dans la colonne, le paramètre de réglage permettant de pondérer les effets de la diffusion par rapport aux effets de la propagation. Plus précisément, le paramètre de réglage, de valeur très faible, notamment très inférieure à 1, permet de pondérer le terme de diffusion.

Le terme de diffusion du modèle de l'invention résulte des échanges entre les phases liquide et gazeuse. En effet, dans une colonne de distillation à garnissages, les flux de gaz, ou vapeur, montants sont en contact avec les flux de liquide descendants. Cela est modélisé simplement par un unique flux de vapeur en contact avec un unique flux de liquide, à travers une interface de contact unique. A l'interface, le liquide et le gaz sont concomitants et satisfont à chaque instant à l'équilibre thermodynamique, ce qui impose les concentrations des composants en phase liquide et en phase gazeuse. Plus on est loin de l'interface, moins les fluides sont couplés thermodynamiquement. Par conséquent, loin de l'interface, les concentrations sont différentes des concentrations à l'interface. Dans chaque phase, des flux de diffusion vers l'interface tendent à rehomogénéiser les concentrations. Grâce à la prise en compte, non seulement du phénomène de convection des composants le long de la colonne, mais également de leur diffusion, le procédé de l'invention fournit une estimation pertinente des concentrations.

En outre, en ramenant l'expression du phénomène de diffusion le long du même axe que celui le long duquel les concentrations varient en raison du terme de propagation, à savoir l'axe longitudinal de la colonne, le poids des calculs à effectuer est réduit et la simulation peut être mise en oeuvre en temps réel.

On peut noter qu'un tel modèle provient d'une analyse microscopique, en particulier d'une mise en équation de certains des phénomènes impliquant la concentration des composants dans une section infinitésimale de la colonne.

Le terme de diffusion du modèle résulte de la sorte, notamment, des échanges microscopiques entre les phases liquide et gazeuse.

De façon la plus courante, les colonnes étant orientées verticalement, la position selon l'axe longitudinal de la colonne représentera la position verticale le long de la colonne. L'invention trouvera cependant aussi ses applications dans des colonnes présentant une autre orientation, notamment horizontale.

A titre d'exemple, le modèle conforme à l'invention délivre la valeur des concentrations selon un axe dont l'origine est placée à l'extrémité de la colonne au niveau de laquelle la concentration du composé le moins volatile est minimale et orienté dans le sens croissant de la concentration dudit composé le moins volatile. Autrement dit, pour des colonnes de distillation d'air, présentant une orientation verticale, le composé le moins volatile est l'oxygène et l'origine est le sommet de la colonne, l'axe étant orienté en suivant la concentration croissante en oxygène, c'est-à-dire, du haut en bas.

Il est à noter que, par colonne de distillation à garnissages, on comprend des colonnes de distillation comprenant des éléments se présentant sous forme de tôles métalliques définissant un réseau de canaux de circulation du liquide et du gaz parcourant la colonne, lesdites tôles étant configurées afin que lesdits canaux soient fortement imbriqués de manière à favoriser une mise en contact des phases liquides et des phases gazeuses circulant dans lesdits canaux. Cela étant, l'invention s'applique plus largement à toute technologie de colonnes de distillation comprenant des éléments définissant un tel réseau de canaux de circulation de fluide.

Avantageusement, le modèle utilise deux échelles de temps différentes pour tenir compte à la fois de la circulation longitudinale et de la circulation selon une direction normale à l'interface des composants dans la section infinitésimale considérée, c'est-à-dire, selon une direction transversale à l'axe longitudinal de la colonne, ladite circulation selon une direction normale à l'interface étant plus rapide que la circulation longitudinale.

Grâce à l'utilisation de ces deux échelles, l'ensemble des phénomènes physiques se produisant dans la colonne est pris en compte dans le modèle tout en permettant des simplifications diminuant le poids des calculs à effectuer.

Selon cet aspect de l'invention, ledit modèle exploite, par exemple, une équation aux dérivées partielles de convection-diffusion liant une dérivée première selon le temps, une dérivée première et une dérivée seconde selon la position longitudinale dans la colonne d'une valeur en relation avec la concentration desdites composants dans la colonne, ledit paramètre de réglage étant associé à ladite dérivée seconde.

Ledit modèle exploite en outre, par exemple, une expression approchée de la concentration desdits composants en fonction d'une valeur intermédiaire issue de la résolution de ladite équation.

Ladite expression approchée est, notamment, un développement limité par rapport au paramètre de réglage, ledit développement limité comportant un terme d'ordre 0, exprimant les phénomènes lents, et un terme d'ordre 1, perturbatif.

On tire de ladite équation aux dérivées partielles un profil, selon le temps et la position longitudinale dans ladite colonne, de ladite valeur intermédiaire et on détermine un profil, selon le temps et la position longitudinale dans ladite colonne, de la concentration des composants dans la colonne, en reportant ladite valeur intermédiaire dans ladite expression approchée.

A titre d'exemple, l'équation aux dérivées partielles a la forme suivante :

$$f(X)\frac{\partial X}{\partial t} = \frac{\partial}{\partial z}\left[-LX + Vk(X)\right] + \epsilon\frac{\partial}{\partial z}\left[G(X)\frac{\partial X}{\partial z}\right]$$

dans laquelle :

- t représente le temps ;
- z représente la position le long de l'axe de la colonne orienté du haut vers le bas ;
- L et V représentent les débits respectifs de liquide et de gaz dans la colonne ;
- X est un vecteur représentant ladite valeur intermédiaire à l'instant t, à la position z ;
- k est une matrice de fonctions, avantageusement non-linéaires, de X exprimant l'équilibre thermodynamique entre les phases liquide et gazeuse des composants ;
- f et G sont des matrices de fonctions de X ; et
- $\epsilon$ est le paramètre de réglage.

Par matrices de fonctions, on entend des applications de l'ensemble $[0\,;1]^A$ des vecteurs à valeurs, réelles, dans l'intervalle $[0\,;1]$, de dimension A, où A est la taille du vecteur X, dans l'ensemble $M(R)_{AxN}$ des matrices à valeur dans l'ensemble des réels R, à A lignes et N colonnes, N valant 1 ou A.

Selon une réalisation préférée, lorsque le mélange contient M composants, la taille du vecteur X est égale à M-1, étant donné que la somme des concentrations des composants est égale à 1.

A titre d'exemple, dans une unité de séparation d'air, si les composants intéressants sont l'oxygène, l'azote et l'argon, la taille du vecteur X est égale à 2. Dans le cas d'un mélange binaire simplifié, par exemple un mélange d'oxygène et d'azote, le vecteur X est de taille 1 et l'équation aux dérivées partielles est alors purement scalaire.

Ledit terme de diffusion est, par exemple, fonction du débit de liquide et de gaz dans la colonne. Autrement dit, dans l'équation donnée plus haut, la matrice de fonctions G est paramétrée par L et V.

Les fonctions f et G pourront dépendre d'un vecteur de paramètres $\sigma$ représentant les retenues de liquide et de gaz.

Selon une réalisation, $\sigma$ et/ou L et/ou V dépendent du temps t et/ou de la position z.

De préférence, le procédé comprend une étape de résolution numérique de l'équation aux dérivées partielles.

A titre d'exemple, cette résolution numérique utilise une technique de différences finies en temps et en espace pour assurer un calcul rapide et une faible complexité de calcul. Selon une réalisation, le pas temporel utilisé pour la résolution numérique est compris entre une valeur de l'ordre de la seconde à une valeur de l'ordre de la minute, environ, et le pas spatial est fixé à 10 centimètres environ.

Le schéma numérique utilisé pour traiter l'équation peut être écrit sous forme implicite ou explicite. Pour disposer de concentrations calculées qui ne soient ni négatives ni supérieures à 1, on pourra préférer l'utilisation d'une méthode de forme implicite, même si elle nécessite plus de calculs.

Selon un mode de réalisation, la colonne comporte des points d'alimentation et/ou de tirage du produit et/ou de tout ou partie des composants du produit. Ledit modèle divise ladite colonne en plusieurs sections, dites sections homogènes, chacune prévue entre deux points d'alimentation et/ou de tirage voisins selon la hauteur de la colonne. Ledit terme de convection et/ou ledit terme de diffusion sont adaptés à chaque section homogène. On pourra en particulier adapter le vecteur de paramètres $\sigma$. Adapter les jeux de paramètres utilisés à chaque section homogène permet d'améliorer la précision du modèle.

Ledit modèle pourra encore exploiter des conditions limites décrivant le principe de conservation de la masse entre deux sections de la colonne, notamment entre deux sections homogènes, et aux extrémités de ladite colonne.

Les conditions limites complètent ainsi le modèle aux extrémités d'une section homogène de la colonne à garnissages. Deux cas se présentent. Le premier cas est celui dans lequel la section est située à une extrémité de la colonne. Dans ce cas, la condition limite traduit un recyclage partiel ou total du flux quittant la colonne pour obtenir de la vapeur à l'extrémité haute de la colonne et du liquide à l'extrémité basse de la colonne. Le deuxième cas est celui dans lequel la section est connectée à une autre section. Dans ce cas, la condition limite traduit un prélèvement ou une injection de liquide et/ou de gaz entre les deux sections adjacentes.

Cela étant, ledit procédé pourra en outre comprendre les étapes de :

- mesure de la concentration d'au moins un desdits composants en au moins un endroit de la colonne ; et
- réglage du modèle à l'aide du paramètre de réglage déterminé à partir de la concentration mesurée.

Plus précisément, selon ledit procédé, on pourra, notamment de façon itérative :

- estimer la concentration dudit composant au niveau dudit endroit de la colonne où la mesure a lieu à l'aide dudit modèle avec une première valeur dudit paramètre de réglage,
- établir une erreur entre la valeur estimée et la valeur mesurée de la concentration,
- établir une seconde valeur du paramètre de réglage en fonction de ladite erreur,
- remplacer la première valeur du paramètre de réglage par la seconde valeur dans ledit modèle.

Ainsi, la comparaison des concentrations mesurées à des endroits déterminés de la colonne, par exemple avec un analyseur de concentrations, et des concentrations déterminées aux mêmes endroits en utilisant le modèle de l'invention fournit des erreurs d'estimation qui sont alors utilisées pour régler le modèle. La concentration mesurée pourra en particulier être une concentration en tête et/ou fond de colonne, lieu de haute pureté de l'un des composants du mélange.

L'invention concerne également un dispositif de détermination des concentrations de composants chimiques d'un produit, notamment de l'air, dans une colonne de distillation, notamment à garnissages, ledit dispositif comprenant un ou plusieurs capteurs, des moyens de mise en oeuvre d'un modèle permettant d'estimer la concentration des composants, à partir de mesures effectuées par le ou les capteurs, ledit modèle exploitant un paramètre de réglage permettant de prendre en compte des variations de fonctionnement de la colonne, caractérisé en ce qu'il comprend en outre des moyens pour détecter les valeurs dudit paramètre de réglage sortant d'une plage de variations nominale dudit paramètre de manière à pouvoir diagnostiquer une défaillance de l'un ou plusieurs desdits capteurs.. Ledit dispositif est en particulier configuré pour la mise en oeuvre du procédé décrit plus haut.

L'invention concerne aussi une unité de séparation d'air comprenant au moins une colonne de distillation d'air et un dispositif de détermination des concentrations de composants de l'air dans la colonne tel que décrit plus haut.

L'invention concerne encore un programme d'ordinateur comportant des instructions pour la mise en oeuvre du procédé évoqué plus haut, lorsque le programme est exécuté par un processeur.

L'invention concerne aussi un support d'enregistrement dans lequel est stocké ledit programme.

On va maintenant décrire des exemples de réalisation de l'invention de façon plus précise, mais non limitative, en regard des dessins annexés sur lesquels :

- la figure 1 est un schéma synoptique illustrant la structure et le fonctionnement d'une unité de séparation d'air selon un mode de réalisation de l'invention ;
- la figure 2 est un schéma illustrant une section infinitésimale d'une colonne de l'unité de séparation de la figure 1 ;
- la figure 3 est un schéma illustrant les phénomènes en jeux dans la section infinitésimale de la figure 3 ;
- la figure 4 est un schéma illustrant le fonctionnement du procédé de détermination de concentrations selon un mode de réalisation de l'invention ;
- la figure 5 illustre un premier exemple de processus de détection d'une défaillance d'un capteur d'une unité de séparation d'air selon le procédé conforme à l'invention, par le biais d'une représentation de la valeur dudit paramètre de réglage en fonction du temps,
- la figure 6 illustre une phase d'apprentissage d'un second exemple de processus de détection d'une défaillance d'un capteur d'une unité de séparation d'air selon le procédé conforme à l'invention, par le biais d'une représentation de la valeur dudit paramètre de réglage en fonction du temps,
- la figure 7 illustre une phase d'exploitation du processus de la figure 6, par le biais d'une représentation de la valeur dudit paramètre de réglage en fonction du temps,
- la figure 8 illustre une phase d'apprentissage d'un troisième exemple de processus de détection d'une défaillance d'un capteur d'une unité de séparation d'air selon le procédé conforme à l'invention, par le biais d'une représentation de la valeur dudit paramètre de réglage en fonction du temps,
- la figure 9 illustre une phase d'exploitation du processus de la figure 8, selon une première variante, par le biais d'une représentation de la valeur dudit paramètre de réglage en fonction du temps,
- la figure 10 illustre une phase d'exploitation du processus de la figure 8, selon une seconde variante, par le biais d'une représentation de la valeur dudit paramètre de réglage en fonction du temps,
- la figure 11 illustre une division en plusieurs zones d'une unité de séparation d'air selon un mode de mise en oeuvre du procédé conforme à l'invention,
- la figure 12 illustre une phase d'apprentissage d'un autre mode de mise en oeuvre du procédé conforme à l'invention, par le biais d'une représentation de la valeur desdits paramètres de réglage, associés à chacune des zones de ladite unité de séparation de la figure 11, en fonction du temps,
- la figure 13 illustre une phase d'exploitation dudit mode de mise en oeuvre, par le biais d'une représentation de la valeur desdits paramètres de réglage, associés à chacune des zones de ladite unité de séparation de la figure 11, en fonction du temps, et
- la figure 14 illustre des états de ladite unité de séparation de la figure 11, en fonction du temps, d'après les observations faites en relation avec la figure 13.

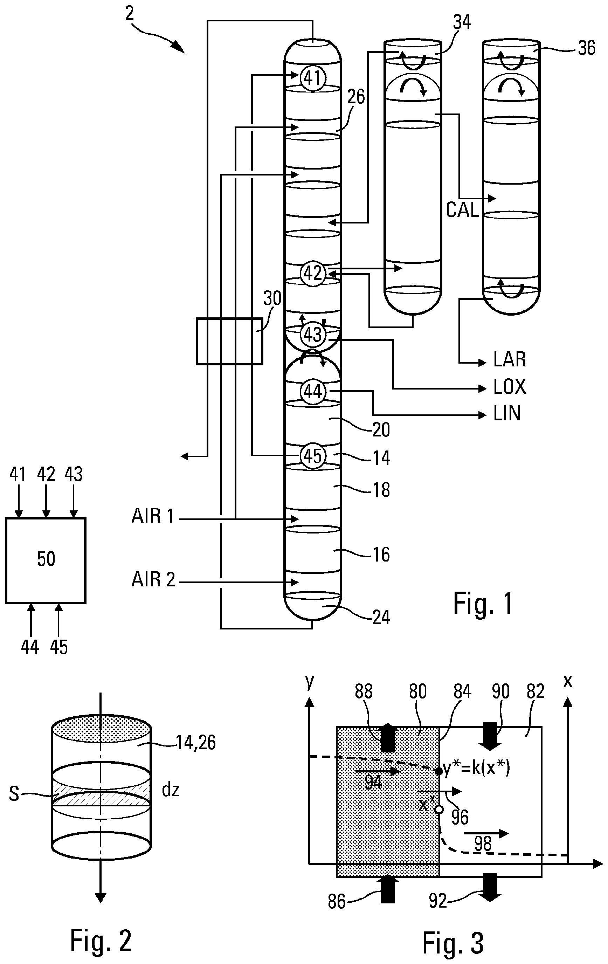

La figure 1 illustre une unité cryogénique 2 de séparation d'air selon un mode de réalisation de l'invention. Cette unité permet d'obtenir de l'oxygène, de l'azote et de l'argon, pratiquement pur, à partir de l'air, que ce soit sous forme liquide ou gazeuse.

De façon connue, ladite unité de séparation 2 comprend une première colonne de distillation 14 à garnissages, dite colonne haute pression, et une seconde colonne de distillation 26 à garnissages, située ici dans la continuité verticale de la colonne haute pression 14. Elle comprend encore une troisième colonne de distillation 34 à garnissages, dite colonne d'argon brut, et une quatrième colonne de distillation 36 à garnissages, dite colonne d'argon pur.

La colonne haute pression 14 comprend plusieurs sections homogènes 16, 18, 20, ici trois. La colonne basse pression 26 comprend également plusieurs sections homogènes, ici cinq. La colonne d'argon brut comprend ici une seule section homogène. La colonne d'argon pur comprend plusieurs sections homogènes, ici deux.

A chaque extrémité d'une section homogène de l'une des colonnes a lieu soit une introduction d'air ou une introduction d'un ou de composants, issus de la distillation dans l'une des autres colonnes, soit un tirage d'un ou de composants issus de la distillation dans la colonne en cause, ceci en phase liquide et/ou en phase gazeuse. On assure de la sorte une distillation du type connu sous le nom de distillation à reflux.

Sans rentrer plus dans le détail, la colonne haute pression 14 est alimentée en air en phase liquide, comme illustré par la flèche associée à la mention AIR 1, et en phase gazeuse, comme illustré par la flèche associée à la mention AIR 2. L'alimentation en air en phase gazeuse est effectuée en fond de colonne et l'alimentation en air sous forme liquide est effectuée au dessus de la section homogène 16 se trouvant juste au dessus du fond 24 de la colonne haute pression 14. De l'azote liquide, quasiment pur, noté LIN sur la figure 1, est récupéré en haut de la colonne haute pression 14. De l'oxygène liquide, quasiment pur, noté LOX, est récupéré en fond 28 de la colonne basse pression 26. Du liquide riche en argon, noté LAR, est récupéré en bas de la colonne d'argon pur 36.

L'unité de séparation 2 pourra en outre comprendre un échangeur de chaleur 30 au niveau duquel certains des flux introduits et/ou tirés des colonnes haute et/ou basse pression 14, 26 échangent de la chaleur. Lesdites colonnes haute et basse pression pourront par ailleurs être configurées pour autoriser un échange de chaleur entre le haut de ladite colonne haute pression 14 et le fond de la dite colonne basse pression 26 afin de respectivement permettre une liquéfaction et une vaporisation des composants se trouvant à ce niveau.

De manière avantageuse, trois analyseurs de la concentration d'oxygène 41, 42, 43 sont placés à des endroits déterminés de la colonne basse pression 26 et deux analyseurs 44, 45 sont placés à des endroits déterminés de la colonne haute pression 14.

Par ailleurs, l'unité de séparation 2 comprend un dispositif de détermination 50 des concentrations des composants chimiques de l'air à tout endroit des colonnes haute pression 14 et basse pression 26. Ce dispositif 50 comprend notamment un processeur permettant l'exploitation d'un modèle décrivant la variation des concentrations des composants en fonction du temps et de la position le long de chacune de ces colonnes 14, 26.

Ledit modèle utilise les mesures effectuées par les analyseurs 41, 42, 43, 44, 45, ceci en exploitant un paramètre de réglage permettant de prendre en compte des variations de fonctionnement de la colonne,

Le fonctionnement de ce dispositif 50 est détaillé dans la suite de la description, en relation avec le mode de réalisation dudit modèle exploitant un terme de propagation en relation avec une convection desdits composants le long de la colonne et un terme de diffusion axiale en relation avec une diffusion desdits composants dans la colonne, le paramètre de réglage permettant de pondérer les effets de la diffusion par rapport aux effets de la propagation.

Dans cette description, le terme colonne sans autre précision désignera l'une des colonnes 14, 26 de la figure 1.

Le modèle utilisé par le dispositif 50 comprend l'équation aux dérivées partielles (1) suivante :

$$f(X)\frac{\partial X}{\partial t} = \frac{\partial}{\partial z}\left[-LX + Vk(X)\right] + \epsilon\frac{\partial}{\partial z}\left[G(X)\frac{\partial X}{\partial z}\right] \quad (1)$$

dans laquelle :

- t représente le temps ;
- z représente la position le long de l'axe de la colonne orienté du haut vers le bas ;
- L et V représentent les débits respectifs de liquide et de gaz dans la colonne ;
- X est un vecteur représentant une valeur intermédiaire liée à la concentration des composants à l'instant t, à la position z ;
- k est une matrice de fonctions exprimant l'équilibre thermodynamique entre les phases liquide et gazeuse des composants ;
- f et G sont des matrices de fonctions de X ; et
- $\epsilon$ est le paramètre de réglage.

La fonction k pourra être non-linéaire. A titre d'exemple, elle s'exprime de la façon suivante :

$$k(x) = \frac{\alpha x}{1 + (\alpha - 1)x}$$

dans laquelle $\alpha$ est la volatilité relative du composant en cause par rapport au composé de rang M dont la concentration n'est pas calculé mais déduite de la concentration calculé des autres composés, comme expliqué plus haut.

Le premier terme de l'équation (1) représente le terme de propagation. Le deuxième terme de l'équation (1) représente le terme de diffusion axiale. Il provient de la prise en compte d'un phénomène microscopique rapide, à savoir la diffusion microscopique transversale, ceci après simplification.

Les origines microscopiques de l'équation 1 vont maintenant être détaillées en référence aux figures 2 et 3. Pour la clarté de la description, le mélange est considéré comme binaire oxygène/azote, de sorte que l'équation 1 est scalaire, x et y correspondant à la concentration d'oxygène, respectivement en phase liquide et en phase gazeuse.

La figure 2 représente une section infinitésimale S de hauteur dz de la colonne 14, 26 dans laquelle sont étudiés les phénomènes de convection et de diffusion.

Dans la colonne de distillation à garnissages 14, 26, les flux de gaz montants sont en contact avec les flux de liquide descendants.

En référence à la figure 3, ce phénomène physique peut être modélisé simplement comme un seul flux de gaz 80 en contact avec un seul flux de liquide 82 à travers une seule interface de contact 84.

Les flèches 86, 88 montrent le déplacement vertical de bas en haut du flux de gaz 80 et les flèches 90, 92 montrent le déplacement vertical de haut en bas du flux de liquide 82, l'axe des abscisses représentant la distance à l'interface liquide/gaz et les axes des ordonnées X et Y représentent les concentrations en liquide et en gaz respectivement.

Au niveau de l'interface 84, le liquide et le gaz sont concomitants et à l'équilibre thermodynamique à tout instant, ce qui impose les concentrations au niveau de l'interface selon la relation (2):

$$y^* = k(x^*) \tag{2}$$

dans laquelle l'astérisque "*" indique qu'il s'agit d'une variable évaluée à l'interface 84.

Loin de l'interface, les fluides ne sont plus thermodynamiquement couplés de sorte que les concentrations sont différentes des concentrations à l'interface.

Le déplacement descendant de liquide représenté par les flèches 90, 92 est décrit par la relation (3) :

$$\sigma_L \frac{\partial x}{\partial t} = -\frac{\partial(Lx)}{\partial z} + \frac{\lambda_L}{\epsilon}(x^* - x) \tag{3}$$

dans laquelle $\sigma_L$ représente la retenue en phase liquide du composant en cause et $\lambda_L$ représente un coefficient de diffusion en phase liquide.

De la même manière, le déplacement ascendant de gaz représenté par les flèches 86, 88 est décrit par la relation (4) :

$$\sigma_V \frac{\partial y}{\partial t} = \frac{\partial(Vy)}{\partial z} + \frac{\lambda_V}{\epsilon}(y^* - y) \tag{4}$$

dans laquelle $\sigma_V$ représente la retenue en phase vapeur du composant en cause et $\lambda_V$ représente un coefficient de diffusion en phase vapeur.

De manière remarquable, ledit modèle ne se contente pas de se placer dans une échelle de temps lente, dans laquelle le phénomène de diffusion radiale permettant un échange de masse entre les phases liquide et gazeuse est négligé car il est trop rapide.

Au contraire, le modèle de l'invention utilise également une échelle rapide pour décrire ce phénomène de circulation représenté par les flèches 94, 96, 98.

Dans chaque phase, des flux de diffusion 94, 98 tendent à rehomogénéiser les concentrations. La diffusion finit alors par affecter l'interface 84 qui ne peut pas accumuler ou créer de la matière. Ainsi, un flux d'échange de masse 96 doit traverser l'interface 84 et permet ainsi de coupler les flux de diffusion de chaque phase. Cet échange de masse entre les 2 phases est exprimé par la relation (5) :

$$\frac{\lambda_V}{\lambda_L \epsilon}\left(Y^* - Y\right) + \frac{1}{\epsilon}\left(X^* - X\right) = 0 \tag{5}$$

Le paramètre de réglage $\epsilon$ est très faible, notamment très inférieur à 1. Le terme $\lambda_V/\epsilon$ est assimilable au coefficient de diffusion associé aux flux de diffusion dans la phase gazeuse, et le terme $\lambda_L/\epsilon$ est assimilable au coefficient de diffusion associé aux flux de diffusion dans la phase liquide. L'hypothèse selon laquelle les coefficients de diffusion sont très grands est raisonnable lorsque le garnissage des colonnes est efficace. Avec cette hypothèse, le système d'équations (2) à (5) peut être simplifié.

Pour réaliser cette simplification, une technique dite variété invariante (« invariant manifold ») est utilisée ici, notamment une technique dite variété centre (« centre manifold »). Cette technique permet de préserver un bilan massique global. Il permet en outre de ne pas rendre une phase prépondérantes par rapport à l'autre dans la structure du modèle, notamment du point de vue des rétentions liquide/vapeur et du point de vue de l'équilibre thermodynamique.

La réduction aboutit alors à l'équation (1), dans laquelle la fonction G permet de relier les conditions de fonctionnement de la colonne aux effets de la diffusion.

La fonction G pourra s'exprimer de la façon suivante :

$$G(X) = \frac{\frac{k'(X)^2}{\lambda_L} + \frac{k'(X)}{\lambda_V}}{(\sigma_L + \sigma_V k'(X))^2}(\sigma_V L + \sigma_L V)^2 \tag{6}$$

dans laquelle k' est la fonction dérivée de la fonction k. Elle permet de mettre en évidence les effets locaux de L et V sur la diffusion.

La fonction f pourra s'exprimer de la façon suivante :

$$f(X) = \sigma_L + \sigma_V k'(X) \tag{7}$$

Il est à noter que les paramètres $\sigma$, L et V pourront dépendre du temps t et de la position z.

Le modèle permet également de décrire les concentrations dans chaque phase.

Plus précisément, on utilise pour cela une expression approchée de la concentration desdits composants en fonction de la valeur intermédiaire issue de la résolution de l'équation (1). Ladite expression approchée est, par exemple, un développement limité par rapport au paramètre de réglage, ledit développement limité comportant un terme d'ordre 0, exprimant les phénomènes lents, et un terme d'ordre 1, perturbatif. On entend par là que le terme d'ordre 0 exprime un fonctionnement du système dans lequel les phénomènes rapides sont considérés comme instantanés et le terme d'ordre 1, perturbatif, prend en compte au moins partiellement la non instantanéité desdits phénomènes rapides.

La concentration x en phase liquide pourra s'exprimer, par exemple, de la façon suivante :

$$x(z, t) = X - \epsilon \sigma_V \frac{G(X)}{\sigma_V L + \sigma_L V} \frac{\partial X}{\partial z} \tag{8}$$

La concentration y en phase gazeuse pourra s'exprimer, par exemple, de la façon suivante :

$$y(z, t) = k(X) + \epsilon \sigma_L \frac{G(X)}{\sigma_V L + \sigma_L V} \frac{\partial X}{\partial z} \tag{9}$$

Ainsi, pour estimer les concentrations en phase liquide et en phase gazeuse d'un composant, on pourra tirer de l'équation (1) un profil, selon le temps et la position verticale dans la colonne, de ladite valeur intermédiaire X puis déterminer un profil, selon le temps et la position verticale dans ladite colonne, de la concentration x en phase liquide et y en phase gazeuse des composants dans la colonne, en reportant ladite valeur intermédiaire X dans ladite expression approchée (8) et/ou (9).

C'est cette approche qui est ici mise en oeuvre dans le dispositif 50.

Outre l'équation (1), le modèle comprend des conditions limites décrivant ici le principe de conservation de la masse entre deux sections, notamment entre deux sections homogènes, de la colonne et aux extrémités de ladite colonne. Plus particulièrement, les effets de la diffusion dans l'équation (1) doivent être préservés à ces endroits limites.

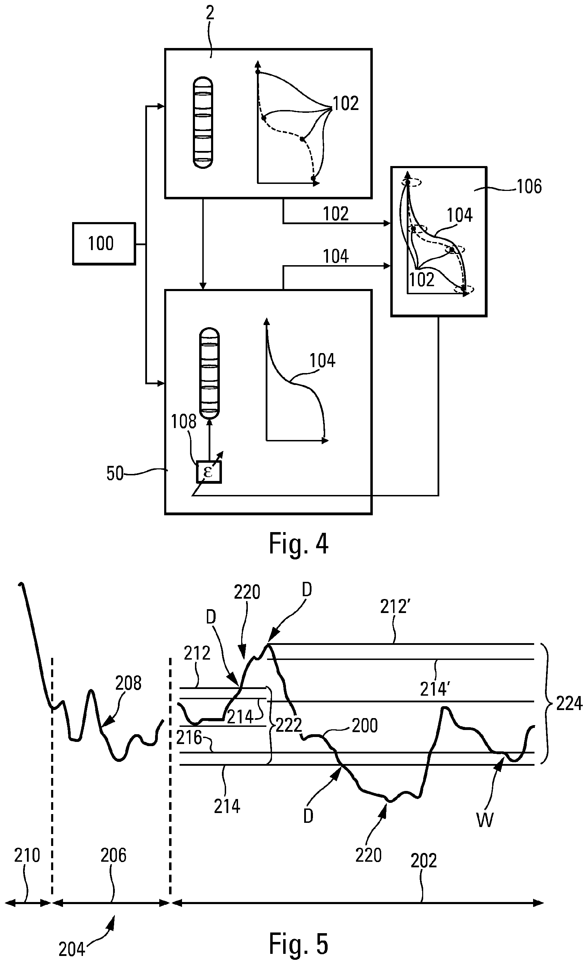

La figure 4 illustre le fonctionnement du dispositif de détermination 50 du profil de concentration en oxygène dans l'unité de séparation d'air 2.

Des données connues 100 sont fournies au dispositif de détermination 50. Il s'agit notamment de températures et/ou pressions et/ou débits de liquide et/ou de gaz à des endroits déterminés de l'unité de séparation 2.

En outre, les analyseurs de concentration 41, 42, 43, 44, 45 fournissent au dispositif de détermination 50 des mesures de concentration discrètes 102 en oxygène dans des endroits déterminés des colonnes 14, 26. On établit ainsi une version initiale du modèle. En variante, on pourra également choisir des valeurs de départ arbitraires.

A partir de ces données, le dispositif de détermination 50 muni du modèle représenté par l'équation (1) estime de manière itérative le profil de concentration en oxygène dans les colonnes 14, 26.

Lors de la première itération, le paramètre de réglage ε est fixé à une certaine valeur. Le dispositif de détermination 50 estime un profil de concentration en oxygène 104 à l'aide du modèle qui lui est incorporé avec cette valeur du paramètre de réglage.

Ensuite, le dispositif de détermination 50 compare les concentrations estimées aux endroits déterminés avec les mesures discrètes et en déduit des erreurs d'estimation (bloc 106 sur la figure 4) qu'il utilise pour adapter le paramètre de réglage $\varepsilon$ (bloc 108 sur la figure 4).

Ainsi, au départ, lors des toutes premières itérations, le profil de concentration peut être très imprécis. Au bout d'un certain temps, le paramètre $\varepsilon$ est correctement réglé. Le dispositif de détermination 50 fournit alors un profil de concentration précis.

De préférence, chaque colonne 14, 26 a son propre paramètre de réglage $\varepsilon$.

Lors de l'estimation du profil de concentrations, le dispositif de détermination 50 résout de manière numérique l'équation aux dérivées partielles (1).

Il utilise pour cela une technique de différences finies en temps et en espace pour assurer un calcul rapide de faible complexité. Le pas temporel choisi pour la résolution numérique est fixé ici à une seconde environ et le pas spatial est fixé à 10 centimètres environ.

Le schéma numérique utilisé pour traiter l'équation est écrit de façon à ce que les concentrations calculées ne soient ni négatives ni supérieures à 1, par exemple à l'aide d'un schéma implicite.

Selon une réalisation préférée, le principe d'adaptation 108 du paramètre de réglage est le suivant :

- si le paramètre de réglage $\varepsilon$ a une valeur correcte, alors le modèle mathématique de l'équation (1) est réaliste. Dans ce cas, les erreurs d'estimation sont nulles ;
- si les erreurs d'estimation ne sont pas nulles, alors le modèle mathématique n'est pas correct. Par conséquent, il est nécessaire de changer la valeur du paramètre de réglage $\varepsilon$.

Ici, le dispositif de détermination 50 utilise une équation supplémentaire (10) :

$$\frac{d\varepsilon}{dt} = M \qquad\qquad (10)$$

dans laquelle M est fonction des erreurs d'estimation et éventuellement d'autres paramètres.

L'équation (10) permet de modifier le paramètre de réglage $\varepsilon$ de manière continue afin de maintenir les erreurs d'estimation aussi faibles que possible.

La fonction M peut, par exemple, utiliser directement une ou plusieurs erreurs d'estimation et peut prendre en compte d'autres paramètres tels que les débits de liquide et/ou de gaz, les pressions, etc.

Une fonction linéaire M simple dépendant uniquement d'une seule erreur d'estimation peut être utilisée. Il est également possible d'utiliser une structure plus complexe pour la fonction M afin d'accélérer la baisse des erreurs d'estimation.

Cela étant, les figures 5 à 10, 12 et 13 illustrent une courbe 200 donnant la valeur du paramètre de réglage $\varepsilon$ en fonction du temps.

Selon l'invention, ledit procédé de détermination des concentrations comprend une étape 202, aussi appelée dans la suite phase d'exploitation, dans laquelle on détecte les valeurs dudit paramètre de réglage sortant d'une plage de variations nominale dudit paramètre. On diagnostique de la sorte une défaillance D de l'un ou plusieurs desdits capteurs, par exemple en émettant une alerte. Ainsi, grâce à l'invention, on profite de l'une des variables déjà utilisées pour obtenir une évaluation de la valeur des concentrations des composants pour en outre détecter une anomalie des capteurs servant à ladite évaluation.

De façon avantageuse, ledit procédé comprend une étape 204 préalable dans laquelle on établit au moins une dite plage de variations nominale dudit paramètre.

Ladite étape préalable 204 pourra comprendre une étape d'apprentissage 206 dans laquelle on enregistre tout ou partie des valeurs prises par ledit paramètre de réglage sur au moins une période de temps, dites valeurs de références 208.

Comme plus particulièrement illustré à la figure 5, ladite étape préalable pourra aussi comprendre une étape d'initialisation 210 pendant laquelle on ignore les valeurs du paramètre de réglage afin d'éviter de le prendre en compte alors qu'il est encore trop imprécis, comme l'éventualité en a été expliquée plus haut.

Ladite étape préalable 204 pourra en outre comprendre une étape de détermination de ladite plage de variations nominale à partir desdites valeurs de référence 206. Il pourra s'agir d'une étape dans laquelle on repère les valeurs minimum et maximum prises par ledit paramètre de réglage. Ladite plage de variation déterminée est ici située entre une valeur maximum autorisée 212, 212' et une valeur minimum autorisée 214.

Une plage intermédiaire située ici entre une valeur de seuil supérieur intermédiaire 214, 214' et une valeur de seuil inférieur intermédiaire 216 pourra également être prévue. Le modèle conforme à l'invention est alors configuré pour émettre un avertissement W lorsque la valeur du paramètre de réglage sort de ladite plage intermédiaire.

Suite à ladite phase préalable, on exploite ledit modèle en surveillant les valeurs prises par ledit paramètre de réglage. On pourra ainsi enregistrer les valeurs prises par ledit paramètre de réglage lors d'une excursion 220 dudit paramètre de réglage en dehors de ladite plage de variations nominale. On pourra en particulier déclencher alors des opérations de vérification des capteurs.

A ce sujet, selon un mode de réalisation de l'invention, ledit procédé pourra comprendre une étape de modification d'une plage de variation nominale, initiale, 222, en une plage élargie 224, tenant compte des valeurs prises par le paramètre de réglage lors de ladite excursion 220 quand la vérification effectuée permet d'établir que la défaillance associée est un faux positif. On entend par faux positif l'émission par le modèle d'une alerte signifiant la présence d'une anomalie alors que l'anomalie, après vérification n'est pas réelle. Autrement dit, tous les capteurs sont en état de marche.

Ici, la valeur maximum autorisée 212 associée à la plage de variation nominale initiale est remplacée par la valeur supérieure autorisée 212' de la plage de variation nominale élargie 224. Il en est de même avec la plage intermédiaire pour laquelle la valeur de seuil supérieur intermédiaire 214 associée à la plage de variation nominale initiale est remplacée par la valeur de seuil supérieure intermédiaire 214' associée à la plage de variation nominale élargie 224.

Autrement dit, à la figure 5, l'excursion 220 intervenant la première dans le temps, par valeurs supérieures, correspond à une étape de mise à jour de la plage de variations nominale dudit paramètre de réglage, seule l'excursion 220 intervenant en second lieu, par valeurs inférieurs, correspondant à la détection d'une défaillance D réelle. On pourra par ailleurs observer que l'avertissement W émis par la suite n'est pas suivi de la détection d'une défaillance, la valeur du paramètre de réglage rentrant postérieurement à l'intérieur de la plage intermédiaire.

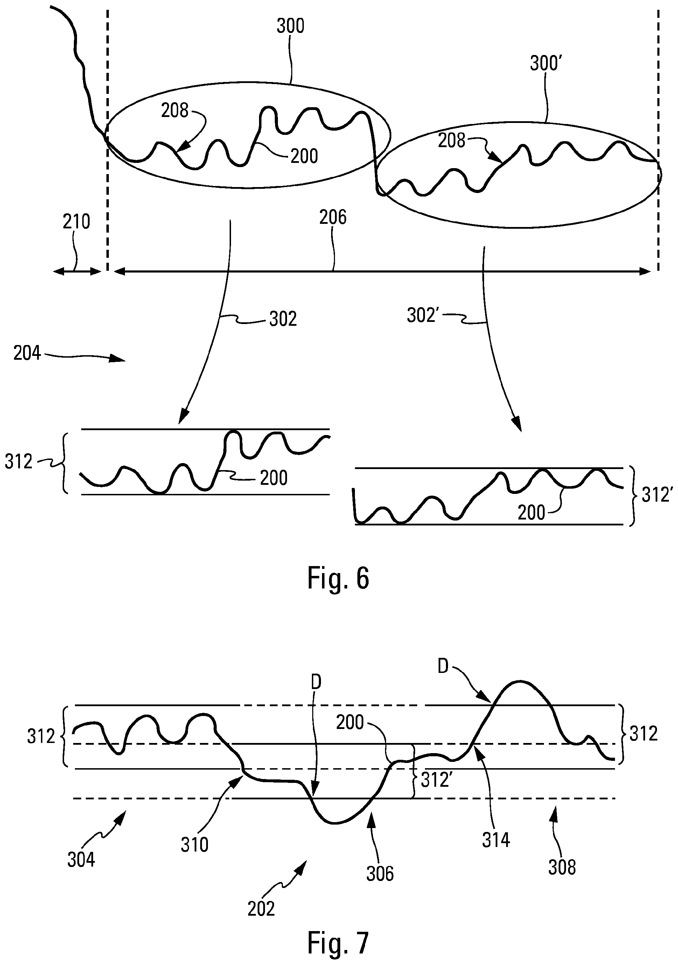

Selon un mode de réalisation avantageux, illustré aux figures 6 et 7, on prévoit plusieurs plages de variations nominale 312, 312', chaque plage correspondant à des conditions de fonctionnement données 300, 300' de ladite colonne de distillation.

Plus précisément, comme illustré à la figure 6, en phase d'apprentissage 206, on détecte différentes conditions de fonctionnement 300, 300' et on associe à chacune desdites conditions de fonctionnement 300, 300' l'une desdites plages de variations nominale 312, 312', comme symbolisé par les flèches illustrées 302, 302'.

Comme illustré à la figure 7, une fois en phase d'exploitation, ledit procédé comprend une étape de détermination des conditions de fonctionnement de la colonne de distillation et une étape de sélection de la plage de variations nominale 312, 312' correspondante, ceci de façon itératif.

On a représenté ici une succession d'une première phase d'exploitation 304 correspondant à une première plage de variations nominale 312, suivie d'une seconde phase d'exploitation 306 correspondant à une seconde plage de variations nominale 312', suivie d'une troisième phase d'exploitation 308 dans laquelle on retrouve la première plage de variations nominale 312.

On constate qu'un tel mode de réalisation évite d'émettre une alerte correspondant à une défaillance alors que la valeur du paramètre de réglage sort, par valeurs inférieures, de la première plage de variations nominale 312 au point 310 car l'on est alors passé dans la seconde phase d'exploitation 306, phase de fonctionnement dans laquelle la valeur du paramètre de réglage se trouve alors dans la plage de variations nominale 312' correspondante. Il est en de même au point 314 quoique la valeur du paramètre de réglage sort cette fois par valeurs supérieures de la seconde plage de variations nominale 312', la première plage de variation nominale 312 étant alors applicable. Les alertes correspondant aux défaillances D émises, ici lors de la seconde 306 et de la troisième 308 phases de fonctionnement, sont alors plus pertinentes car tenant compte des différentes conditions de fonctionnement de la colonne.

Selon une première variante, correspondant aux figures 5, 6 et 7 déjà commentées, ladite étape d'apprentissage 206 est opérée avec ladite colonne de distillation, seulement.

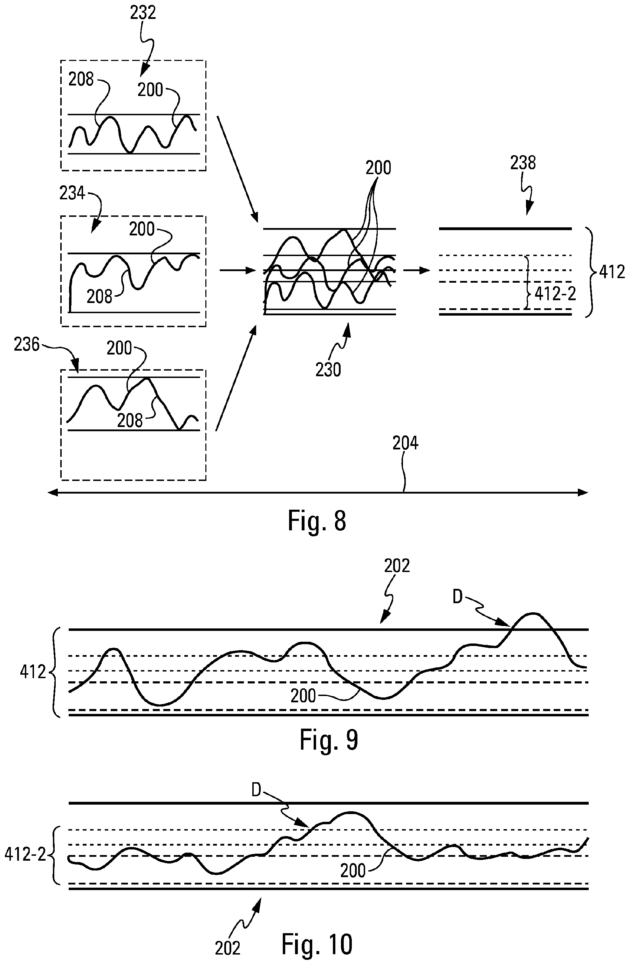

Selon une autre variante, illustrée à la figure 8, ladite étape préalable 204 comprend une étape 230 de fusion de données 232, 234, 236 provenant de plusieurs unités ou colonnes de distillation et ladite étape de détermination de ladite plage de variations nominale, dite alors globale, exploite ladite fusion de données. Ladite étape préalable comprend alors une étape 238 de sélection de ladite plage de variations nominale parmi les plages de variations nominales correspondant à chacune desdites unités et la plage de variation nominale globale 412. Ici seule la plage de variations nominale 412-2 correspondant aux données 234 de la seconde unité a été repérée et illustrée dans un souci de facilité de représentation.

On dispose de la sorte de plusieurs plages de variations nominale possibles, à savoir, par exemple, ladite plage de variations nominale globale 412, prévue comme étant la plus large, et des plages de variations nominales 412-2 correspondant à tout ou partie des unités ou colonnes de distillation dont proviennent les données. On peut de la sorte écourter, voire se passer de la phase d'apprentissage en utilisant l'historique de certaines installations, en particulier les installations réputées de fonctionnement proche ou similaire.

Comme illustré aux figures 9 et 10, en phase d'exploitation on choisit alors l'une desdites plages de variations nominale pour exploiter ledit modèle. A la figure 9, la plage de variations nominale globale 412 a été choisie. A la figure 10, il s'agit de la plage 412-2 correspondant aux données de la seconde exploitation. On constate de la sorte la différence sur l'émission des alertes D.

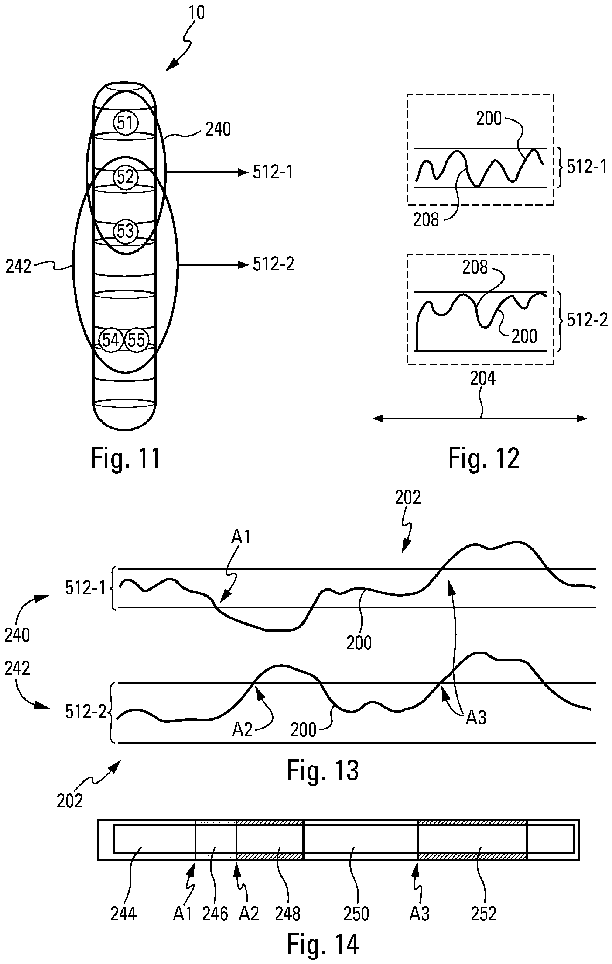

Comme illustré à la figure 11, l'on pourra considérer que l'unité de distillation 10 comprend plusieurs zones 240, 242 exploitant chacune un dit paramètre de réglage. Plus particulièrement, chacune des zones comprend des capteurs ou analyseurs de concentration 51, 54, 55 distincts, certaines desdites zones pouvant avoir des capteurs ou analyseurs de concentration 52, 53 communs. Il est ici prévu une première zone 240 contenant les premier, second et troisième capteurs 51, 52, 53 et une second zone 242 contenant les second, troisième, quatrième et cinquième capteurs 52, 53, 54, 55.

Comme illustré à la figure 12, on détermine une dite plage de variations nominale 512-1, 512-2 amenée à être utilisée pour chacune desdites zones 240, 242 de la colonne 10 en phase d'exploitation.

Comme illustré aux figures 13 et 14, un intérêt d'une telle solution est de faciliter l'identification du ou des capteurs défaillants.

A la figure 13, on voit qu'une première alerte A1 est émise correspondant à la première zone 240 suivie d'une seconde alerte A2 correspondant à la seconde zone 242, cette seconde alerte A2 arrivant postérieurement à la première A1.

A la figure 14, ceci se traduit par une première séquence 244 précédent la première alerte A1, première séquence correspondant à l'émission d'une information d'absence de défaillance. Cette première séquence 244 est suivie d'une seconde séquence 246 allant de la première alerte A1 à la seconde alerte A2, seconde séquence pendant laquelle une information de défaillance probable est émise. Cette seconde séquence 246 est alors suivie d'une troisième séquence 248 intervenant après la seconde alerte A2, troisième séquence pendant laquelle une information de défaillance est émise. L'analyse de la succession des séquences 244, 246, 248 permet de penser que c'est l'un des capteurs de la première zone 240 qui est touché puisque l'anomalie est d'abord apparue dans cette zone.

Si l'on se reporte de nouveau à la figure 13, on constate que, après un retour à la normale, une nouvelle alerte A3 est émise simultanément pour les deux zones 240, 242.

A la figure 14, ledit retour à la normale correspond à la quatrième séquence 250 et la nouvelle alerte A3 au début d'une sixième séquence 252 dans laquelle une information de défaillance est directement émise, sans passer par une étape d'émission d'une information de défaillance probable. Cette succession de séquences 250, 252 permet de penser que l'anomalie touche les capteurs 52,53 communs au deux zones 240, 242 puisqu'elle est apparue simultanément dans celles-ci.

Le dispositif de détermination des concentrations conforme à l'invention évoqué plus haut sera bien sûr configuré pour permettre de telles détections de défaillance. Il comprend en ce sens des moyens pour détecter les valeurs 200 dudit paramètre de réglage sortant d'une plage de variations nominale dudit paramètre. Lesdits moyens pourront exploiter pour cela un programme d'ordinateur exécuté, par exemple, par le processeur 50 dudit dispositif. Ledit programme d'ordinateur est éventuellement stocké sur un support d'enregistrement.

## Revendications

1. Procédé de détermination des concentrations de composants chimiques d'un produit, notamment de l'air, dans une colonne de distillation, procédé dans lequel on met en oeuvre un modèle permettant d'estimer la concentration des composants à partir de mesures effectuées par un ou plusieurs capteurs, ledit modèle exploitant un paramètre de réglage permettant de prendre en compte des variations de fonctionnement de la colonne, **caractérisé en ce qu'**il comprend une étape dans laquelle on détecte les valeurs (200) dudit paramètre de réglage sortant d'une plage de variations nominale dudit paramètre pour diagnostiquer une défaillance de l'un ou plusieurs desdits capteurs.

2. Procédé selon la revendication 1 comprenant une étape préalable (204) dans laquelle on établit au moins une dite plage de variations nominale dudit paramètre.

3. Procédé selon la revendication 2 dans laquelle ladite étape préalable (204) comprend une étape d'apprentissage (206) dans laquelle on enregistre tout ou partie des valeurs prises par ledit paramètre de réglage sur au moins une période de temps, dites valeurs de références (208).

4. Procédé selon la revendication 3 dans lequel ladite étape préalable (204) comprend une étape de détermination (210) de ladite plage de variations nominale à partir desdites valeurs de référence (208).

5. Procédé selon la revendication 4 dans lequel ladite étape préalable (204) comprend une étape (230) de fusion de données provenant de plusieurs colonnes de distillation et ladite étape de détermination de ladite plage de variations nominale exploite ladite fusion de données pour déterminer une plage de variations nominale globale (412).

**6.** Procédé selon l'une quelconque des revendications précédentes comprenant une étape d'enregistrement de valeurs prises par ledit paramètre de réglage lors d'une excursion (220) dudit paramètre de réglage en dehors de ladite plage de variations nominale, dite initiale (212), et une étape de modification de la plage de variation nominale initiale en une plage élargie (212'), tenant compte des valeurs prises par le paramètre de réglage en dehors de ladite plage de variations nominale initiale (212), quand une vérification permet d'établir que la défaillance associée à ladite excursion n'existe pas.

**7.** Procédé selon l'une quelconque des revendications précédentes dans lequel on prévoit plusieurs plages de variation nominale (312, 312'), chaque plage correspondant à des conditions de fonctionnement données (300, 300') de ladite colonne de distillation et ledit procédé comprend une étape de détermination des conditions de fonctionnement (300, 300') de la colonne de distillation et une étape de sélection de la plage de variations nominale (312, 312') correspondante.

**8.** Procédé selon l'une quelconque des revendications précédentes dans lequel ladite colonne de distillation comprend plusieurs zones (240, 242) exploitant chacune un dit paramètre de réglage, procédé dans lequel on utilise une dite plage de variations nominale (512-1, 512-2) pour chacune desdites zones (240, 242).

**9.** Procédé selon l'une quelconque des revendications précédentes dans lequel ledit modèle exploite un terme de propagation en relation avec une convection desdits composants le long de la colonne et un terme de diffusion axiale en relation avec une diffusion desdits composants dans la colonne, le paramètre de réglage permettant de pondérer les effets de la diffusion par rapport aux effets de la propagation.

**10.** Procédé selon l'une quelconque des revendications précédentes, comprenant en outre les étapes de :

- mesure de la concentration d'au moins un desdits composants en au moins un endroit de la colonne ; et
- réglage du modèle à l'aide du paramètre de réglage déterminé à partir de la concentration mesurée.

**11.** Procédé selon la revendication 10 dans lequel :

- on estime la concentration dudit composant au niveau dudit endroit de la colonne où la mesure a lieu à l'aide dudit modèle avec une première valeur dudit paramètre de réglage,
- on établit une erreur entre la valeur estimée et la valeur mesurée de la concentration,
- on établit une seconde valeur du paramètre de réglage en fonction de ladite erreur,
- on remplace la première valeur du paramètre de réglage par la seconde valeur dans ledit modèle.

**12.** Dispositif de détermination des concentrations de composants chimiques d'un produit, notamment de l'air, dans une colonne de distillation (10, 14, 26), ledit dispositif comprenant un ou plusieurs capteurs (41-45, 51-55), des moyens de mise en oeuvre d'un modèle permettant d'estimer la concentration des composants, à partir de mesures effectuées par le ou les capteurs, ledit modèle exploitant un paramètre de réglage permettant de prendre en compte des variations de fonctionnement de la colonne, **caractérisé en ce qu'**il comprend des moyens pour détecter les valeurs dudit paramètre de réglage sortant d'une plage de variations nominale dudit paramètre de manière à pouvoir diagnostiquer une défaillance de l'un ou plusieurs desdits capteurs.

**13.** Unité de séparation d'air (2) comprenant au moins une colonne de distillation d'air (10, 14, 26) et un dispositif de détermination des concentrations de composants de l'air dans la colonne (10, 14, 26) selon la revendication 12.

**14.** Programme d'ordinateur comportant des instructions pour la mise en oeuvre du procédé selon l'une quelconque des revendications 1 à 11, lorsque le programme est exécuté par un processeur (50).

**15.** Support d'enregistrement lisible par ordinateur dans lequel est stocké le programme selon la revendication 14.

**Patentansprüche**

**1.** Verfahren zum Bestimmen der Konzentrationen von chemischen Komponenten eines Produkts, vor allem von Luft, in einer Destillationssäule, Verfahren, bei dem man ein Modell anwendet, das es ermöglicht, die Konzentration der Komponenten ausgehend von Messungen zu schätzen, die durch einen oder mehrere Sensoren durchgeführt werden, wobei das Modell einen Einstellparameter nutzt, der es ermöglicht, Betriebsvariationen der Säule zu berücksichtigen, **dadurch gekennzeichnet, dass** es einen Schritt umfasst, bei dem man die Werte (200) des Einstellparameters, die aus einem nominalen Variationsbereich des Parameters hervorgehen, detektiert, um eine Fehlleistung des einen oder mehrerer der Sensoren zu diagnostizieren.

2. Verfahren nach Anspruch 1, einen vorbereitenden Schritt (204) umfassend, bei dem man mindestens einen nominalen Variationsbereich des Parameters festlegt.

3. Verfahren nach Anspruch 2, wobei der vorbereitende Schritt (204) einen Lernschritt (206) umfasst, bei dem man alle oder einen Teil der Werte speichert, die durch den Einstellparameter über mindestens einen Zeitraum hinweg erhoben werden, die Referenzwerte (208) genannt werden.

4. Verfahren nach Anspruch 3, wobei der vorbereitende Schritt (204) einen Schritt zum Bestimmen (210) des nominalen Variationsbereichs aus den Referenzwerten (208) umfasst.

5. Verfahren nach Anspruch 4, wobei der vorbereitende Schritt (204) einen Schritt (230) zur Fusion von Daten umfasst, die aus mehreren Destillationssäulen stammen und der Schritt zum Bestimmen des nominalen Variationsbereichs die Fusion von Daten nutzt, um einen globalen nominalen Variationsbereichs (412) zu bestimmen.

6. Verfahren nach einem der vorstehenden Ansprüche, einen Schritt zum Speichern von Werten umfassend, die durch den Einstellparameter bei einem Exkurs (220) des Einstellparameters außerhalb des nominalen Variationsbereichs erhoben werden, der ursprünglicher Bereich (212) genannt wird, und einen Schritt zum Ändern des ursprünglichen nominalen Variationsbereichs in einen erweiterten Bereich (212'), unter Berücksichtigung der Werte, die durch den Einstellparameter außerhalb des ursprünglichen nominalen Variationsbereichs (212) erhoben werden, wenn eine Überprüfung eine Festlegung ermöglicht, dass es die dem Exkurs zugeordnete Fehlleistung nicht gibt.

7. Verfahren nach einem der vorstehenden Ansprüche, wobei man mehrere nominale Variationsbereiche (312, 312') vorsieht, wobei jeder Bereich gegebenen Betriebsbedingungen (300, 300') der Destillationssäule entspricht, und das Verfahren einen Schritt zum Bestimmen der Betriebsbedingungen (300, 300') der Destillationssäule und einen Schritt zum Auswählen des entsprechenden nominalen Variationsbereichs (312, 312') umfasst.

8. Verfahren nach einem der vorstehenden Ansprüche, wobei die Destillationssäule mehrere Zonen (240, 242) umfasst, die jeweils einen Einstellparameter nutzen, Verfahren, bei dem man einen nominalen Variationsbereich (512-1, 512-2) für jede der Zonen (240, 242) verwendet.

9. Verfahren nach einem der vorstehenden Ansprüche, wobei das Modell ein Ausbreitungsende im Verhältnis mit einer Konvektion der Komponenten entlang der Säule und ein axiales Diffusionsende im Verhältnis mit einer Diffusion der Komponenten in der Säule nutzt, wobei es der Einstellparameter ermöglicht, die Auswirkungen der Diffusion im Verhältnis zu den Auswirkungen der Ausbreitung zu gewichten.

10. Verfahren nach einem dervorstehenden Ansprüche, weiter die Schritte umfassend:

  - Messen der Konzentration mindestens einer der Komponenten an mindestens einer Stelle der Säule; und
  - Einstellen des Modells mithilfe des Einstellparameters, der aus der gemessenen Konzentration bestimmt wird.

11. Verfahren nach Anspruch 10, wobei:

  - man die Konzentration der Komponente im Bereich der Stelle der Säule, wo die Messung stattfindet, mithilfe des Modells mit einem ersten Wert des Einstellparameters schätzt,
  - man einen Fehler zwischen dem Schätzwert und dem Messwert der Konzentration festlegt,
  - man einen zweiten Wert des Einstellparameters in Abhängigkeit von dem Fehler festlegt,
  - man den ersten Wert des Einstellparameters durch den zweiten Wert in dem Modell ersetzt.

12. Vorrichtung zum Bestimmen der Konzentrationen von chemischen Komponenten eines Produkts, vor allem von Luft, in einer Destillationssäule (10, 14, 26), wobei die Vorrichtung einen oder mehrere Sensoren (41-45, 51-55) umfasst, Mittel zum Anwenden eines Modells, das es ermöglicht, die Konzentration der Komponenten ausgehend von Messungen zu schätzen, die durch einen oder mehrere Sensoren durchgeführt werden, wobei das Modell einen Einstellparameter nutzt, der es ermöglicht, Betriebsvariationen der Säule zu berücksichtigen, **dadurch gekennzeichnet, dass** es Mittel zum Detektieren der Werte des Einstellparameters umfasst, die aus einem nominalen Variationsbereich des Parameters hervorgehen, um eine Fehlleistung des einen oder mehrerer der Sensoren diagnostizieren zu können.

13. Einheit zum Trennen von Luft (2), mindestens eine Luftdestillationssäule (10, 14, 26) und eine Vorrichtung zum Bestimmen der Konzentrationen von Komponenten der Luft in der Säule (10, 14, 26) nach Anspruch 12 umfassend.

14. Computerprogramm, Befehle zum Anwenden des Verfahrens nach einem der Ansprüche 1 bis 11 umfassend, wenn das Programm von einem Prozessor (50) ausgeführt wird.

15. Von einem Computer lesbares Speichermedium, in dem das Programm nach Anspruch 14 gespeichert ist.


**Claims**

1. A method for the determination of the concentrations of chemical components of a product, in particular of air, in a distillation column; a method wherein a model enabling the estimation of the concentration of components from measurements performed by one or several sensors is implemented; said model using an adjustment parameter enabling the taking into consideration of variations in the operation of the column, **characterised in that** it comprises a step wherein the values (200) of said adjustment parameter outside a nominal range of variations of said parameter are detected for the diagnosis of a fault in said one or several sensors.

2. A method as claimed in claim 1, comprising a preliminary step (204) wherein at least one said nominal range of variations of said parameter is established.

3. A method as claimed in claim 2, wherein said preliminary step (204) comprises a learning step (206) wherein all or part of the values taken by said adjustment parameter over at least one period of time, classified as reference values (208) are recorded.

4. A method as claimed in claim 3, wherein said preliminary step (204) comprises a step (210) for the determination of said nominal range of variations from said reference values (208).

5. A method as claimed in claim 4, wherein said preliminary step (204) comprises a step (230) for the fusion of data originating from several distillation columns, and said step for the determination of said nominal range of variations uses said fusion of data to determine an overall nominal range of variations (412).

6. A method as claimed in any of the preceding claims, comprising a step for the recording of values taken by said adjustment parameter during an excursion (220) of said adjustment parameter outside said nominal range of variations, denominated initial (212), and a step for the modification of the initial nominal range of variations to form an extended range (212'), taking into account the values taken by the adjustment parameter outside said initial nominal range of variations (212), when a verification enables confirmation that the fault associated with said excursion does not exist.

7. A method as claimed in any of the preceding claims, wherein several nominal ranges of variations (312, 312') are foreseen, each range corresponding to given operating conditions (300, 300') of said distillation column and said method comprising a step of determining the operating conditions (300, 300') of the distillation column and a step for the selection of the corresponding range of variations (312, 312').

8. A method as claimed in any of the preceding claims, wherein said distillation column comprises several zones (240, 242), each one using one said adjustment parameter; a method in which one said nominal range of variations (512-1, 512-2) is used for each of said zones (240, 242).

9. A method as claimed in any of the preceding claims, wherein said model uses a propagation term related to the convection of said components throughout the length of the column, and an axial diffusion term related to a diffusion of said components within the column, the adjustment parameter enabling the balancing of the effects of the diffusion against the effects of the propagation.

10. A method as claimed in any of the preceding claims, further comprising the stages of:

measurement of the concentration of at least one of said components in at least one location within the column, and

adjustment of the model by means of the adjustment parameter determined from the concentration measured.

11. A method as claimed in claim 10, wherein:

the concentration of said component is estimated at the level of said location in the column where the measurement took place, by means of said model, with a first value of the said adjustment parameter,
an error is established between the estimated value and the measured value of the concentration,
a second value of the adjustment parameter is established in accordance with said error,
the first value of the adjustment parameter in said model is replaced by the second value.

12. A device for the determination of the concentrations of chemical components of a product, in particular of air, in a distillation column (10, 14, 26); said device comprising one or several sensors (41-45, 51-55); means of implementing a model enabling the estimation of the concentration of the components from measurements performed by the sensor or sensors, said model using an adjustment parameter enabling the taking into consideration of variations in the operation of the column, **characterised in that** it comprises means for the detection of the values of said adjustment parameter outside a nominal range of variations of said parameter to enable the diagnosis of a fault in one or several of said sensors.

13. An air separation unit (2) comprising at least one air distillation column (10, 14, 26) and a device for the determination of the concentrations of the components of the air within the column (10, 14, 26), as claimed in claim 12.

14. A computer programme consisting of instructions for the implementation of the method, as claimed in any of claims 1 to 11, when the programme is executed by a processor (50).

15. A computer-readable recording medium wherein the programme as claimed in claim 14 is stored.

Fig. 1

Fig. 2

Fig. 3

Fig. 4

Fig. 5

Fig. 6

Fig. 7

Fig. 8

Fig. 9

Fig. 10

10

51
52
53

54 55

240

512-1

242

512-2

**Fig. 11**

200

512-1

208

208

512-2

200

204

**Fig. 12**

202

A1

512-1

240

242

512-2

200

A2   200

A3

**Fig. 13**

202

244   A1   246   A2   248   250   A3   252

**Fig. 14**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0701186 A

- US 5313800 A